(19) Europäisches Patentamt
European Patent Office
Office européen des brevets

(11) Veröffentlichungsnummer: **0 290 919**
**A1**

(12) # EUROPÄISCHE PATENTANMELDUNG

(21) Anmeldenummer: 88107063.5

(22) Anmeldetag: 03.05.88

(51) Int. Cl.4: C07C 29/92 , C07C 33/05 , C07C 33/042 , C07C 33/12 , C07C 33/14

(30) Priorität: 13.05.87 DE 3715889
28.07.87 DE 3724899

(43) Veröffentlichungstag der Anmeldung:
17.11.88 Patentblatt 88/46

(84) Benannte Vertragsstaaten:
AT BE CH DE ES FR GB IT LI NL SE

(71) Anmelder: BAYER AG

D-5090 Leverkusen 1, Bayerwerk(DE)

(72) Erfinder: Wintel, Thomas, Dr.
Viktoriastrasse 44
D-5600 Wuppertal 1(DE)
Erfinder: Franckowiak, Gerhard, Dr.
Henselweg 10
D-5600 Wuppertal 1(DE)
Erfinder: Samaan, Samir, Dr.
Paul-Ehrlich-Strasse 21
D-5600 Wuppertal 1(DE)
Erfinder: Heine, Hans-Georg, Dr.
Am Heckerhof 14
D-4150 Krefeld(DE)

(54) **Optisch aktive Octinole und ihre Verwendung bei der Herstellung von Prostaglandinen.**

(57) Die Erfindung betrifft optisch aktive Oktinole der Formeln

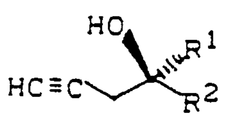

(VIII)

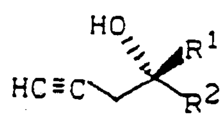

(IX)

in denen $R^1$ und $R^2$ die in der Beschreibung angegebene Bedeutung haben, deren Herstellung und deren Verwendung. Sie eignen sich als Zwischenprodukte zur Herstellung von Prostaglandinen.

EP 0 290 919 A1

## Optisch aktive Octinole und ihre Verwendung bei der Herstellung von Prostaglandinen

Die Erfindung betrifft optisch aktive Octinole, ein Verfahren zu ihrer Herstellung, Permethrinsäureester der Octinole und die Verwendung der Octinolpermethrinsäureester sowie der Octinole bei der Herstellung von Prostaglandinen, insbesondere von Rioprostil und Butaprost.

Die Prostaglandinderivate der Formel I und II sind in den Patenten DE-PS 2 902 699 (= US-PS 4 132 738) genannt.

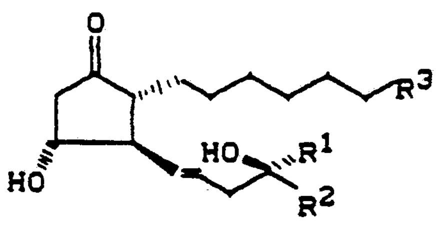

I

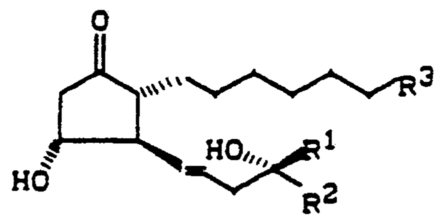

II

$R^1$ = -H, -Me,
$R^2$ = -Alkyl, verzweigtes Alkyl, insbesondere $C_1$-$C_4$-Alkyl, alkyliert-Cycloalkyl, insbesondere $C_1$-$C_4$-alkyliertes $C_3$-$C_7$-Cycloalkyl,
$R^3$ = $-CH_2OH$, $-CO_2-CH_3$.

Das in den genannten Patenten beschriebene Verfahren zur Herstellung der Wirkstoffe I und II ist in dem folgenden Formelschema 1 dargestellt:

Schema 1

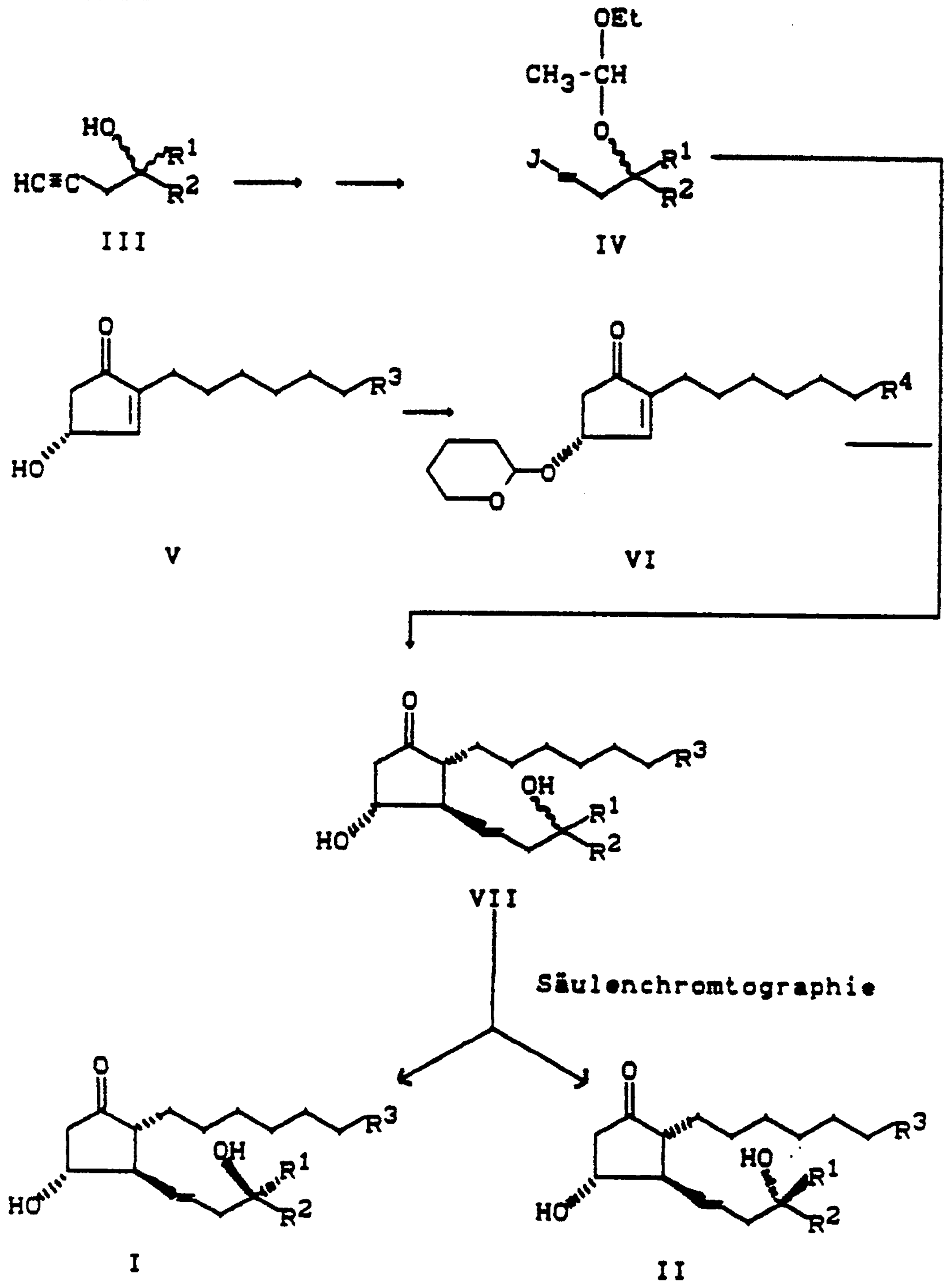


III IV V VI VII I II

Das Verfahren benötigt zur Darstellung der reinen Diastereomeren I bzw. II eine aufwendige säulenchromatographische Auftrennung des Diastereomerengemisches VII. Weiterhin verliert man bei dieser Trennung etwa die Hälfte der eingesetzten, aufwendig herzustellenden Einsatzstoffe III und V in Form der jeweils unerwünschten Diastereomeren.

Diese Nachteile des beschriebenen Verfahrens können umgangen werden durch die Darstellung der optisch aktiven Alkohole VIII und IX durch Racematspaltung von III (Schema 2). Die aus den enantiomerenreinen Alkoholen VIII und IX hergestellten Wirkstoffe I und II fallen in höherer Ausbeute und höherer Reinheit an.

Schema 2

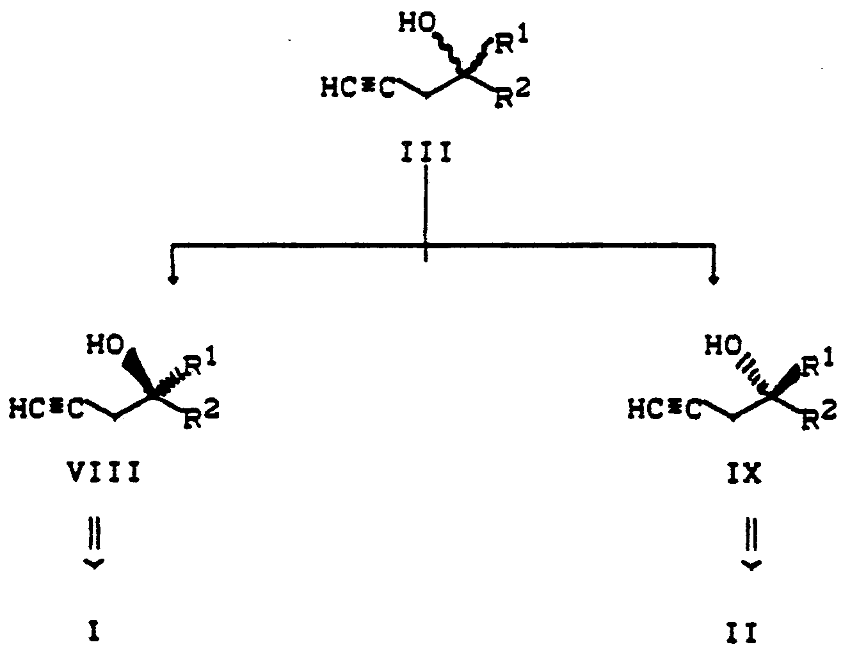

III

VIII

IX

I

II

Die Spaltung von racemischen Alkoholen in ihre Enantiomeren durch Umsetzen mit optisch aktiven Säuren und Ausnützung der Energieunterschiede, die sich in unterschiedlichen physikalischen Eigenschaften der so entstandenen diastereomeren Ester äußern, ist eine seit langem gebräuchliche Methode.

Ebenfalls ist die kinetische Racematspaltung von racemischen Alkoholen über die entsprechenden Ester mit Enzymen lange bekannt.

In der Praxis jedoch ergeben sich bei beiden Methoden vielfältige und unvorhersehbare Schwierigkeiten, die eine generelle Übertragbarkeit der Ergebnisse einer erfolgreichen Racematspaltung selbst auf "ähnliche" Trennprobleme unmöglich macht. Jedes Trennproblem erfordert eine individuelle Lösung. In diesem Sinn bemerkt auch Eliel in "Stereochemie der Kohlenstoffverbindungen", Verlag Chemie Weinheim 1966, S. 59 bis 99: "Racematspaltung ist nach wie vor eine Kunst".

Gegenstand der Erfindung sind nun drei Verfahren (Schema 3), aus den racemischen Alkoholen III unter Verwendung der neuen Zwischenstufen XIII bis XX, die optisch aktiven Alkohole VIII und IX in hoher optischer Reinheit herzustellen.

Hierin bedeuten $R^1$ Wasserstoff oder Methyl und $R^2$ Alkyl, bevorzugt $C_1$-$C_4$-Alkyl, das verzweigt oder geradkettig sein kann oder alkyliertes, bevorzugt $C_1$-$C_4$-alkyliertes Cycloalkyl, insbesondere Cyclobutyl.

Scheme 3

Verfahren 1 | Verfahren 2 | Verfahren 3

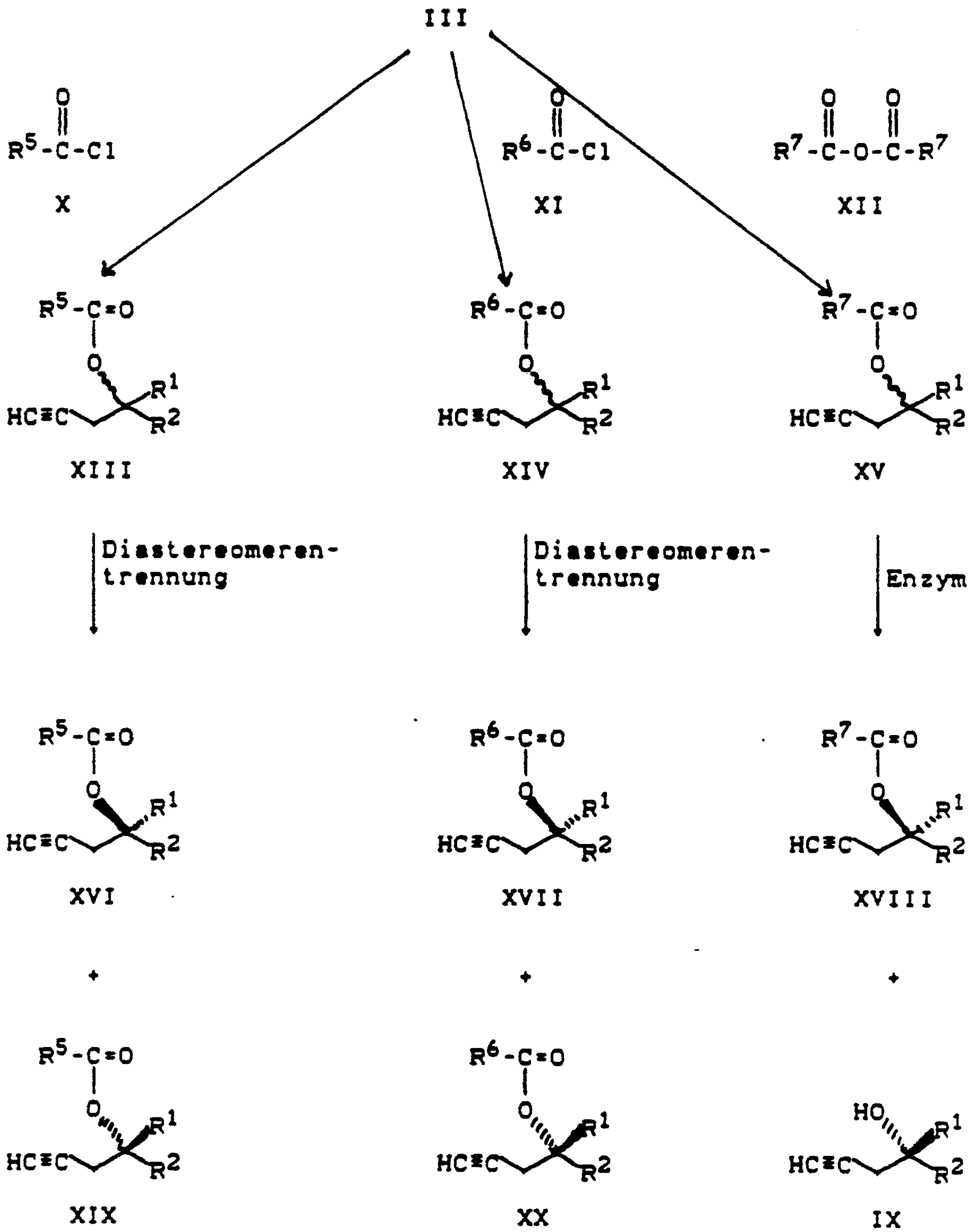


III

X | XI | XII

XIII | XIV | XV

XVI | XVII | XVIII

+ | + | +

XIX | XX | IX

$R^5$ = 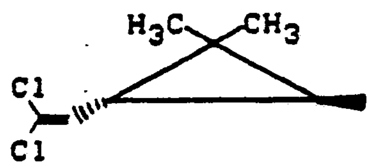 $R^6$ = 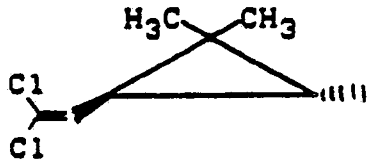

$R^7$ = $-CH_3$, $-C_2H_5$, $-n-C_3H_7$, $-C_6H_5$.

XVI oder XVII oder XVIII → VIII

XIX oder XX → IX

Verfahren 1

Die Darstellung der diastereomeren Ester XIII aus den racemischen Alkoholen III gelingt mit dem 1S-Permethrinsäurechlorid X (EP-PS 00 229 72) oder einem anderen aktivierten Derivat der 1S-Permethrinsäure wie z.B. dem Anhydrid in Gegenwart eines Säurefängers wie z.B. Pyridin, Triethylamin. Gegebenenfalls kann der Reaktionsmischung noch ein Katalysator wie z.B. 4-Dimethylaminopyridin, Diazabicycloundecan, Diazabicyclononen oder Diazabicyclooctan zugesetzt werden. Die Reaktion kann in einem inerten organischen Lösungsmittel wie z.B. Methylenchlorid, Chloroform, Toluol, Xylol, Diethylether, Diisopropylether, Methyl-tert.-butylether, Tetrahydrofuran, Ethylenglykoldimethylether, Diethylenglykoldimethylether, Hexan, Petrolether oder ohne Lösungsmittel bei 10°C bis 120°C, bevorzugt bei 40°C bis 80°C in 1 bis 24 Stunden, bevorzugt 4 bis 16 Stunden, bevorzugt bei Normaldruck durchgeführt werden. Das Rohprodukt kann entweder direkt weiterverarbeitet werden oder durch Filtration in einem inerten Lösungsmittel über Kieselgel gereinigt werden.

Die Diastereomerentrennung gelingt durch präparative Säulenchromatographie an Kieselgel oder an modifizierten Kieselgelen, z.B. RP-18-oder RP-4-Materialien oder durch Flüssig-Flüssig-Verteilung. Bevorzugt wird die Trennung durch fraktionierte Kristallisation aus einem inerten organischen Lösungsmittel wie z.B. Methanol, Ethanol, Isopropanol, Petrolether usw. bevorzugt aus Methanol bei Temperaturen zwischen -70°C bis +40°C, bevorzugt zwischen -20°C bis +20°C innerhalb von 1 bis 48 Stunden, bevorzugt innerhalb von 4 bis 36 Stunden durchgeführt. Zur Verbesserung der Kristallisation können Impfkristalle zugefügt werden. Das Diastereomere XVI erhält man angereichert im Kristallisat und das Diastereomere XIX erhält man angereichert in der Mutterlauge. Das Kristallisat kann in seiner Diastereomerenreinheit verbessert werden, indem man es in wenig Lösungsmittel bei -70°C bis +40°C, bevorzugt bei -20°C bis +20°C einmal oder mehrmals ausrührt oder indem man es einmal oder mehrmals umkristallisiert.

Die optisch aktiven Alkohole VIII oder IX erhält man, indem man die angereicherten Ester XVI oder XIX in einem inerten Lösungsmittel wie z.B. Methanol, Ethanol, Isopropanol, Wasser, Toluol usw. mit starken Basen wie z.B. mit Natriumhydroxid, Kaliumhydroxid, Bariumhydroxid, Calciumhydroxid usw., bevorzugt mit Kaliumhydroxid behandelt. Man läßt hierbei 1 bis 24 Stunden, bevorzugt 6 bis 16 Stunden lang bei 20°C bis 100°C, bevorzugt bei 40°C bis 60°C, bevorzugt bei Normaldruck rühren und extrahiert die Alkohole VIII oder IX mit einem inerten Lösungsmittel wie z.B. Methylenchlorid, Hexan, Petrol ether, Toluol, Xylol usw. aus dem Reaktionsgemisch. Nach Abdampfen des Lösungsmittels erhält man die Alkohole VIII oder IX, die entweder roh weiterverarbeitet werden können oder durch Destillation gereinigt werden können.

Nach Ansäuern der extrahierten Reaktionslösung erhält man die 1S-Permethrinsäure XXI, die über das Säurechlorid X wieder in eine erneute Racematspaltung eingesetzt werden kann.

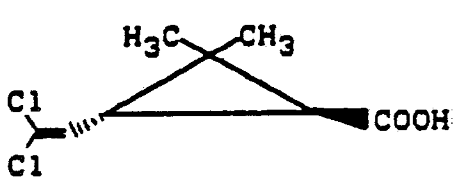

XXI

Bei der alkalischen Esterspaltung kommt es weder bei den Alkoholen VIII bzw. IX noch bei der Permethrinsäure XXI zu einer Racemisierung.

Verfahren 2

Das Verfahren 2 läuft analog zu dem Verfahren 1, nur daß hierbei das 1R-Permethrinsäurechlorid XI (EP-PS 00 229 72) eingesetzt wird. Bei der Kristallisation fällt nun das Diastereomere XX in dem Kristallisat und das Diastereomere XVII bevorzugt in der Mutterlauge an.

Die bei der Verseifung der angereicherten Ester anfallende 1R-Permethrinsäure XXII kann ebenfalls über das Säurechlorid XI erneut in einem Racematspaltung eingesetzt werden.

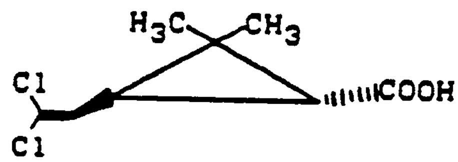

XXII

Verfahren 3

Die Darstellung der Ester XV aus den racemischen Alkoholen III gelingt mit den Anhydriden XII oder anderen aktivierten Carbonsäurederivaten wie z.B. den Säurechloriden in Gegenwart eines Säurefängers wie z.B. Pyridin oder Triethylamin. Ein Überschuß an Säurefänger von 20 bis 30 % stört nicht. Gegebenenfalls kann ein Katalysator, z.B. 4-Dimethylaminopyridin, Diazabicyclopyridin, Diazabicycloundecen, Diazabicyclononen oder Diazabicyclooctan zugesetzt werden. Bevorzugt wird Acetanhydrid oder Acetylchlorid eingesetzt. Die Reaktion kann in einem inerten organischen Lösungsmittel wie z.B. Methylenchlorid, Chloroform, Toluol, Xylol, Diethylether, Diisopropylether, Methyl-tert.-butylether, Tetrahydrofuran, Ethylenglykoldimethylether, Diethylenglykoldimethylether, Hexan, Petrolether oder ohne Lösungsmittel bei 0°C bis 120°C, bevorzugt bei 20°C bis 80°C, in 0,5 bis 24 Stunden, bevorzugt in 1 bis 16 Stunden bevorzugt unter Normaldruck durchgeführt werden. Das Reaktionsprodukt kann entweder direkt weiterverarbeitet werden oder durch Destillation oder durch Extraktion oder durch Filtration in einem inerten Lösungsmittel über Kieselgel gereinigt werden.

Die racemischen Ester XV können in einem Phosphatpuffer von pH 5 bis 9, bevorzugt von pH 6 bis 8 bei 10°C bis 50°C, bevorzugt bei 20°C bis 40°C mit einem Enzym, bevorzugt einer Lipase verseift werden. Ist die Hälfte des Esters XV umgesetzt worden, extrahiert man das Gemisch aus Ester XVIII und Alkohol IX mit einem inerten organischen Lösungsmittel, z.B. Hexan, Petrolether, Toluol, Xylol, Diethylether, Diisopropylether, Methyl-tert.-butylether, Methylenchlorid, Chloroform, Ethylacetat, Methylacetat o.ä. aus der Reaktionslösung und entfernt das Lösungsmittel z.B. durch Vakuumdestillation. Das Produktgemisch aus XVIII und IX kann nun durch Kolonnendestillation, durch präparative Säulenchromatographie oder durch Flüssig-Flüssig-Extraktion in seine Komponenten aufgetrennt werden.

Die Ester XVIII kann man analog zu Verfahren 1 alkalisch verseifen und erhält die Alkohole VIII.

Die über die drei beschriebenen Verfahren hergestellten, optisch aktiven Akohole VIII und IX fallen in hoher chemischer und optischer Ausbeute an. Die beschriebenen Reaktionen lassen sich auch unter technischen Bedingungen problemlos durchführen.

Aus den optisch aktiven Alkoholen VIII und IX lassen sich nun die diastereomerenreinen Prostaglandinderivate I und II in höherer Ausbeute und in höherer Reinheit herstellen.

Bevorzugter Gegenstand der Erfindung sind die drei Verfahren zur Racematspaltung der folgenden Alkohole:

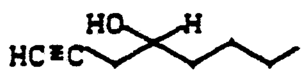

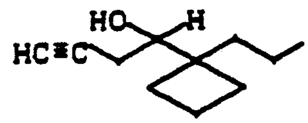

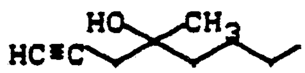

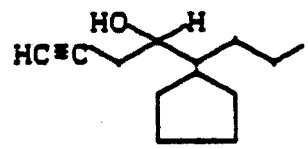

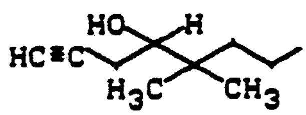

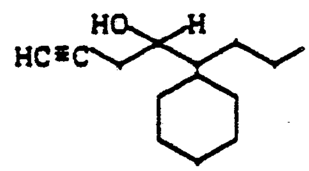

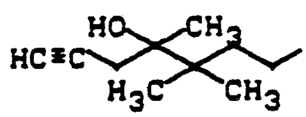

Besonders bevorzugter Gegenstand der Erfindung sind die drei Verfahren zur Racematspaltung der folgenden Alkohole:

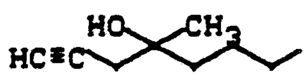

XXIII

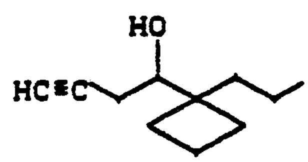

XXIV

Verfahren 1

Die Darstellung der diastereomeren Ester XXV bzw. XXVI aus den racemichen Alkoholen XXIII bzw. XIV gelingt mit dem 1S-Permethrinsäurechlorid X (EP-PS 00 229 72) oder einem anderen aktivierten Derivat der 1S-Permethrinsäure XXI wie z.B. dem symmetrischen Anhydrid, indem man die Reaktanden im Molverhältnis (XXIII bzw. XXIV:X) 1:0,5 bis 1:3, bevorzugt 1:0,8 bis 1:2 mischt.

XXIII 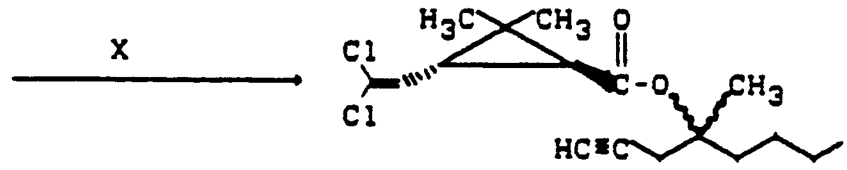


XXV

XXIV 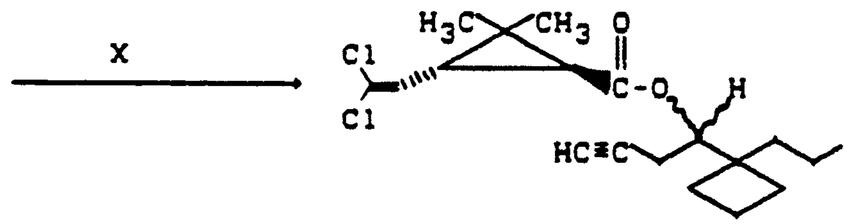


XXVI

Der Zusatz von Säurefängern wie z.B. Pyridin oder Triethylamin im Molverhältnis 1:1 bis 1:10, bevorzugt 1:1 bis 1:3 (bez. X) kann die Reaktion beschleunigen. Gegebenenfalls kann der Reaktionsmischung noch ein Katalysator wie z.B. 4-Dimethylaminopyridin, Diazabicycloundecen, Diazabicyclononen oder Diazabicyclooctan zugesetzt werden.

Die Reaktion kann in einem inerten organischen Lösungsmittel wie z.B. Methylenchlorid, Chloroform, Toluol, Xylol, Diethyleher, Diisopropylether, Methyl-tert.-butylether, Tetrahydrofuran, Ethylenglykoldimethylether, Diethylenglykoldimethylether, Hexan, Petrolether usw. oder ohne Lösungsmittel bei 10°C bis 120°C, bevorzugt bei 40°C bis 80°C in 1 bis 24 Stunden, bevorzugt 4 bis 16 Stunden bevorzugt unter Normaldruck durchgeführt werden.

Nach beendeter Reaktion entfernt man gegebenenfalls auftretende Salze durch Waschen mit Wasser oder durch Filtration und erhält die rohen Ester XXV bzw. XXVI nach Entfernen des Lösungsmittels, z.B. durch Vakuumdestillation. Die Rohprodukte können entweder direkt weiterverarbeitet werden oder durch Filtration in einem inerten Lösungsmittel über Kieselgel oder Aluminiumoxid gereinigt werden.

Die Diastereomerentrennung der Ester XXV bzw. XXVI kann durch präparative Säulenchromatographie an Kieselgel (z.B. Dioxan/Hexan) oder an modifizierten Kieselgelen wie z.B. RP-18-oder RP4-Materialien oder durch Flüssig-Flüssig-Verteilung (Cyclohexan/Nitromethan) oder durch selektive Kristallisation aus einem inerten organischen Lösungsmittel wie z.B. Methanol, Ethanol, Isopropanol, Petrolether usw., bevorzugt aus Methanol durchgeführt werden.

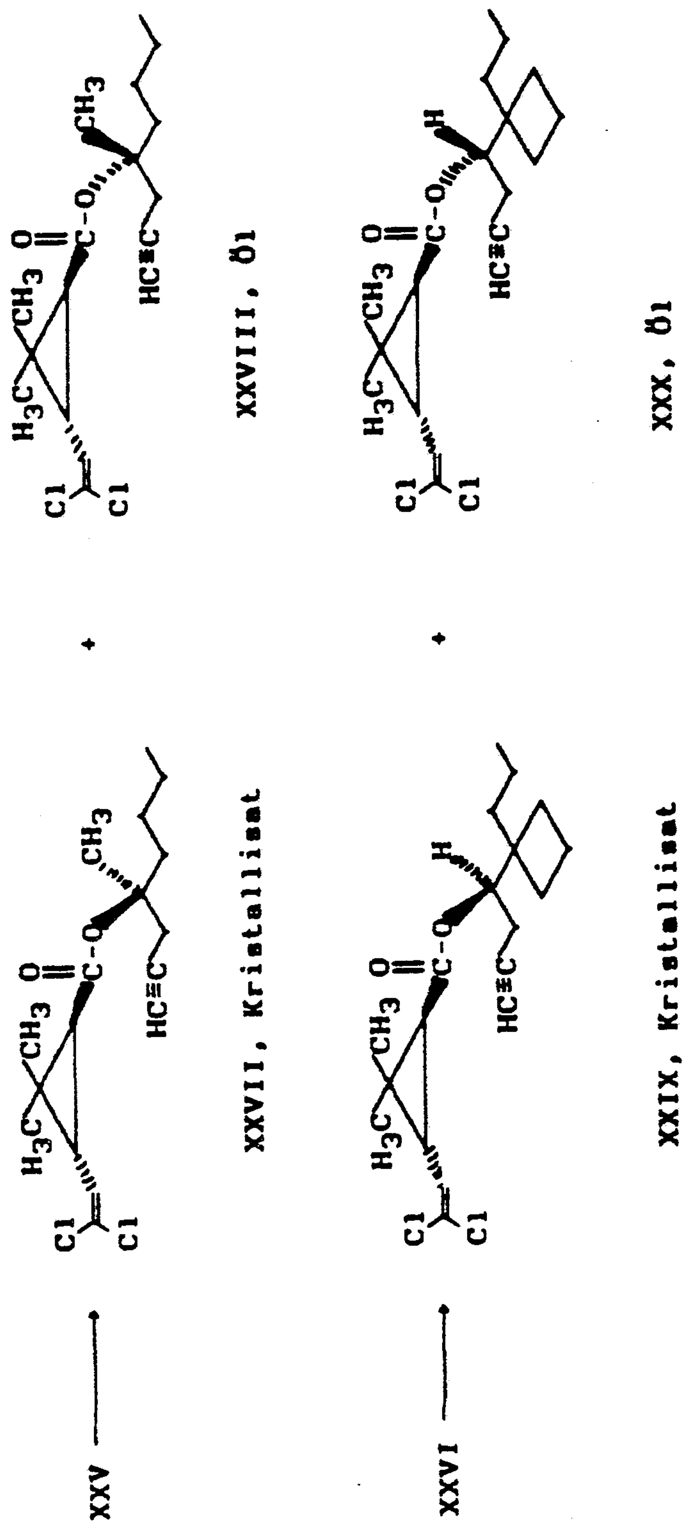

XXV → XXVII, Kristallisat + XXVIII, Öl

XXVI → XXIX, Kristallisat + XXX, Öl

Man läßt zwischen -70°C bis +40°C, bevorzugt zwischen -30°C bis +20°C innerhalb von 1 bis 48 Stunden, bevorzugt 4 bis 36 Stunden, gegebenenfalls unter Zusatz von Impfmaterial kristallisieren. Durch Filtration erhält man die Diastereomeren XXVII bzw. XXIX als Kristallisat und die Diastereomeren XXVIII bzw. XXX nach Entfernen des Lösungsmittels als Öle aus der Mutterlauge.

Das Kristallisat kann in seiner Diastereomerenreinheit verbessert werden, indem man es in wenig Lösungsmittel bei -70°C bis +40°C, bevorzugt bei -30°C bis +20°C einmal oder mehrmals ausrührt oder indem man es einmal oder mehrmals umkristallisiert.

Die optisch aktiven Alkohole XXXI bis XXXIV erhält man, wenn man die diastereomer angereicherten Ester XXVII, XXVIII, XXIX oder XXX in einem inerten Lösungsmittel wie z.B. Methanol, Ethanol, Isopropanol,

Toluol, Wasser usw. mit starken Basen wie Alkali-bzw. Erdalkalihydroxiden, vorzugsweise mit Natriumhydroxid oder Kaliumhydroxid behandelt (Molverhältnis Ester:Base zwischen 1:1 bis 1:10, vorzugsweise 1:1 bis 1:4).

XXVII

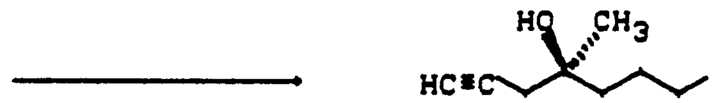

XXXI

XXVIII

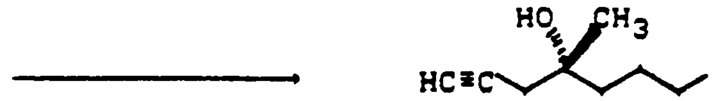

XXXII

XXIX

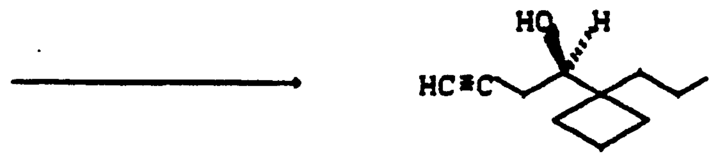

XXXIII

XXX

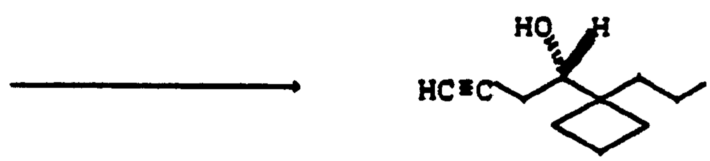

XXXIV

Man rührt die Reaktionslösung 1 bis 24 Stunden, vorzugsweise 4 bis 16 Stunden bei 20°C bis 100°C, vorzugsweise bei 40°C bis 60°C bevorzugt unter Normaldruck, entfernt gegebenenfalls das Lösungsmittel und extrahiert die Alkohole XXXI bis XXXIV mit einem inerten Lösungsmittel wie z.B. Methylenchlorid, Hexan, Petrolether, Toluol, Xylol usw. aus dem Produktgemisch. Nach Entfernen des Lösungsmittels erhält man die rohen Alkohole XXXI bis XXXIV, die entweder roh weiterverarbeitet werden oder durch Destillation gereinigt werden können.

Nach Ansäuern des extrahierten Produktgemisches erhält man nach Extraktion mit einem inerten Lösungsmittel und nachfolgender Entfernung des Lösungsmittels oder durch Filtration die 1S-Permethrinsäure XXI, die über das Säurechlorid X wieder in eine erneute Racematspaltung eingesetzt werden kann.

Bei der alkalischen Esterspaltung kommt es weder bei den optisch aktiven Alkoholen XXXI bis XXXIV noch bei 1S-Permethrinsäure XXI zu einer Racemisierung.

Verfahren 2

Die Darstellung der optisch aktiven Alkohole XXXI bis XXXIV nach Verfahren 2 verläuft analog zu der des Verfahrens 1, nur daß hierbei das 1R-Permethrinsäurechlorid XI (EP-PS 00 229 72) eingesetzt wird.

Aus den racemischen Alkoholen XXIII bzw. XXIV werden zunächst die racemischen Ester XXXV bzw. XXXVI analog hergestellt.

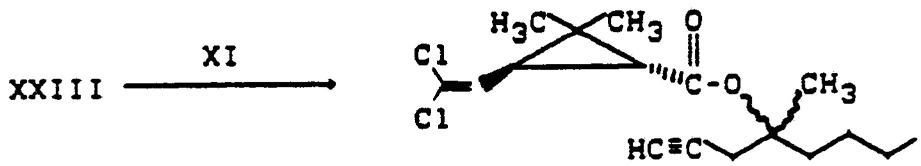


XXXV

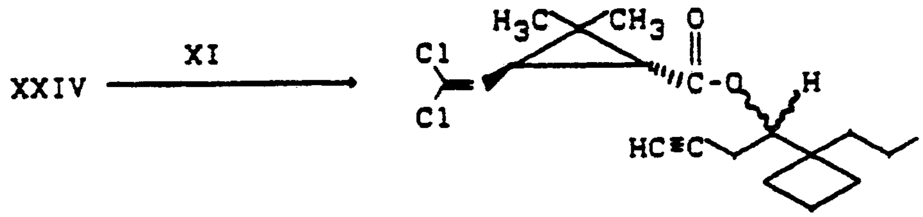


XXXVI

Diese liefern dann analog per Säulenchromatographie oder per Flüssig/Flüssig-Verteilung oder per selektiver Kristallisation die angereicherten Ester XXXVII bis XXXX, wobei die Diastereomeren XXXVIII bzw. XXXX aus dem Kristallisat und die Diastereomeren XXXVII bzw. XXXIX als Öle aus der Mutterlauge erhalten werden können.

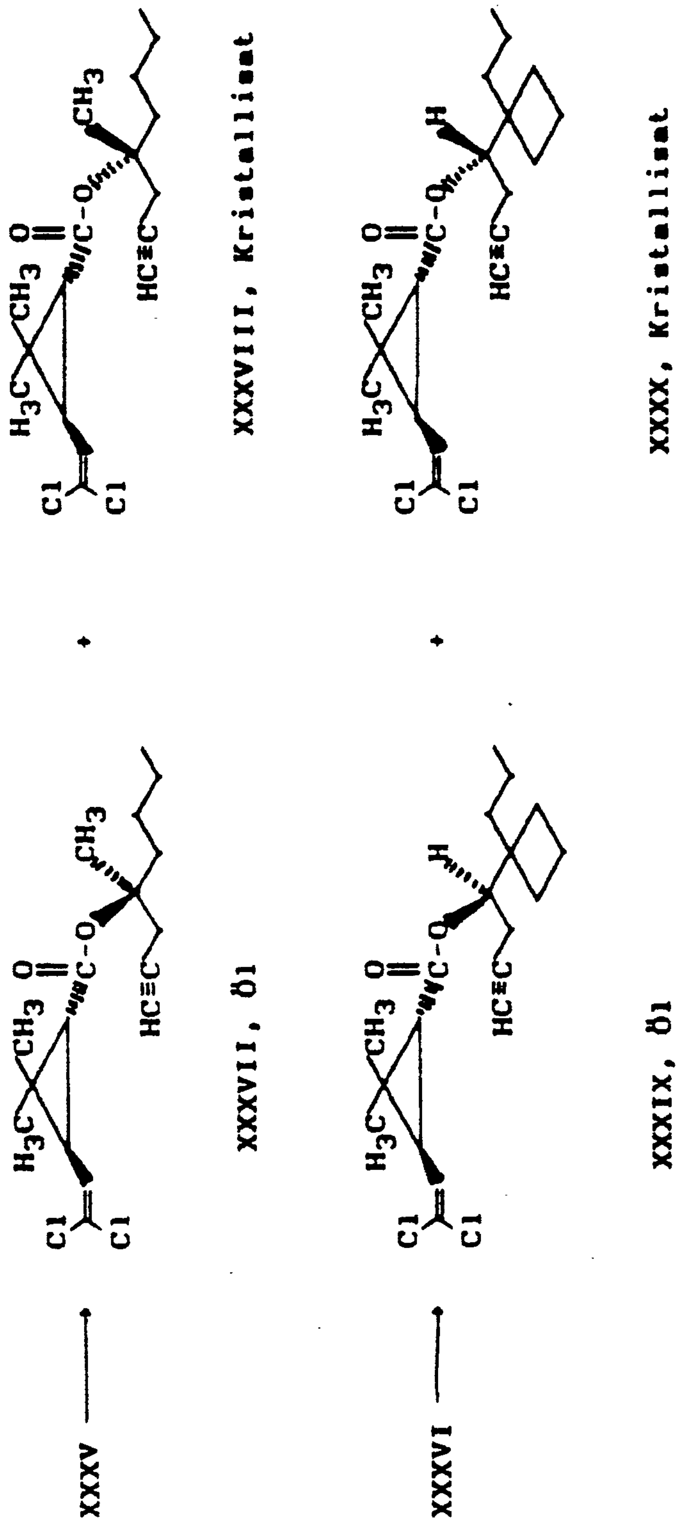

XXXV

XXXVII, Öl

XXXVIII, Kristallisat

XXXVI

XXXIX, Öl

XXXX, Kristallisat

Die angereicherten Ester XXXVII bis XXXX liefern dann analog durch Verseifung die optisch aktiven Alkohole XXXI bis XXXIV.

XXXVII → XXXI
XXXVIII → XXXII
XXXIX → XXXIII
XXXX → XXXIV

Verfahren 3

Die Darstellung der racemischen Ester XXXXI bzw. XXXXII aus den racemischen Alkoholen XXIII bzw. XXIV gelingt mit den Anhydriden XII oder anderen aktivierten Carbonsäurederivaten wie z.B. den Säurechloriden, indem man die Reaktanden im Molverhältnis (XXIII bzw XXIV:XII) 1:0,5 bis 1:3, bevorzugt 1:0,8 bis 1:2 mischt.

XXIII

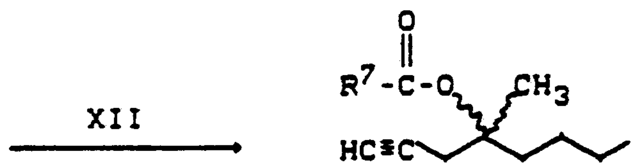


XXXXI

XXIV

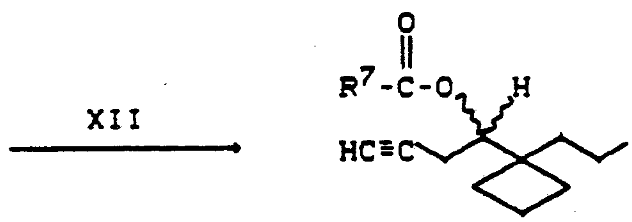


XXXXII

$R^7$ = $-CH_3$, $-C_2H_5$, $-n-C_3H_7$, $-C_6H_5$.

Der Zusatz von Säurefängern wie z.B. Pyridin oder Triethylamin im Molverhältnis 1:1 bis 1:10, vorzugsweise 1:1 bis 1:3 (bez. XII) kann die Reaktion beschleunigen. Gegebenenfalls kann der Reaktionsmischung noch ein Katalysator wie z.B. 4-Dimethylaminopyridin, Diazabicycloundecen, Diazabicyclononen oder Diazabicyclooctan zugesetzt werden.

Die Reaktion kann in einem inerten organischen Lösungsmittel wie z.B. Methylenchlorid, Chloroform, Toluol, Xylol, Diethylether, Diisopropylether, Methyl-tert.-butylether, Tetrahydrofuran, Ethylenglykoldimethylether, Diethylenglykoldimethylether, Hexan, Petrolether usw. oder ohne Lösungsmittel bei 0°C bis 120°C, bevorzugt bei 20°C bis 80°C in 0,5 bis 24 Stunden, bevorzugt 1 bis 16 Stunden bevorzugt unter Normaldruck durchgeführt werden.

Nach beendeter Reaktion entfernt man gegebenenfalls auftretende Salze durch Waschen mit Wasser oder durch Filtration und erhält die rohen Ester XXXXI bzw. XXXXII nach Entfernen des Lösungsmittels z.B. durch Vakuumdestillation. Die Rohprodukte können entweder direkt weiterverarbeitet werden oder durch Filtration in einem inerten Lösungsmittel über Kieselgel oder Aluminiumoxid oder durch Vakuumdestillation gereinigt werden.

Die racemischen Ester XXXXI bzw. XXXXII werden in einem Phosphatpuffer von pH 5 bis 9, bevorzugt pH 6 bis 8 bei 10°C bis 50°C, bevorzugt bei 20°C bis 40°C mit einem Enzym, bevorzugt einer Lipase verseift. Ist die Hälfte des eingesetzten Esters umgesetzt, extrahiert man das Gemisch aus Ester und Alkohol mit einem inerten organischen Lösungsmittel wie z.B. Hexan, Petrolether, Toluol, Xylol, Diethylether, Diisopropylether, Methyl-tert.-butylether, Methylenchlorid, Chloroform, Ethylacetat, Methylacetat o.ä. aus der Reaktionslösung.

XXXXI 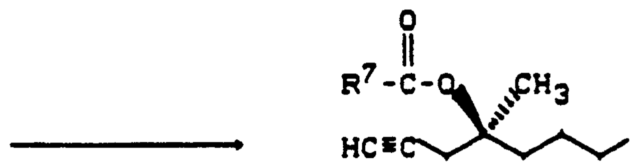 + XXXII

XXXXIII

XXXXII 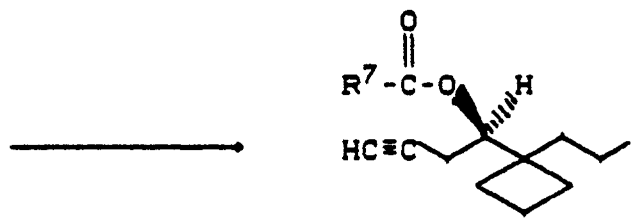 + XXXIV

XXXXIV

$R^7$ = $-CH_3$, $-C_2H_5$, $-n-C_3H_7$, $-C_6H_5$.

Das Produktgemisch aus XXXXIII und XXXII bzw. XXXXIV und XXXIV kann nun durch Kolonnendestillation oder durch präparative Säulenchromatographie oder durch Flüssig-Flüssig-Extraktion in seine Komponenten aufgetrennt werden.

Die optisch aktiven Alkohole XXXI bzw. XXXIII lassen sich aus den optisch aktiven Estern XXXXIII bzw. XXXXIV durch alkalische Verseifung analog, wie im Verfahren 1 beschrieben, darstellen.

XXXXIII → XXXI

XXXXIV → XXXIII

Die über die drei beschriebenen Verfahren hergestellten, optisch aktiven Alkohole XXXI, XXXII, XXXIII und XXXIV fallen in hoher chemischer und optischer Ausbeute an.

Die dargestellten Reaktionen lassen sich auch in technischem Maßstab problemlos durchführen.

Die durch die Racematspaltung hergestellten Alkohole XXXI bis XXXIV liefern nun über optisch aktive Zwischenstufen (Schema 4) die diastereomerenreinen Prostaglandinderivate XXXXV und XXXXVI in höherer Reinheit und in höherer Ausbeute.

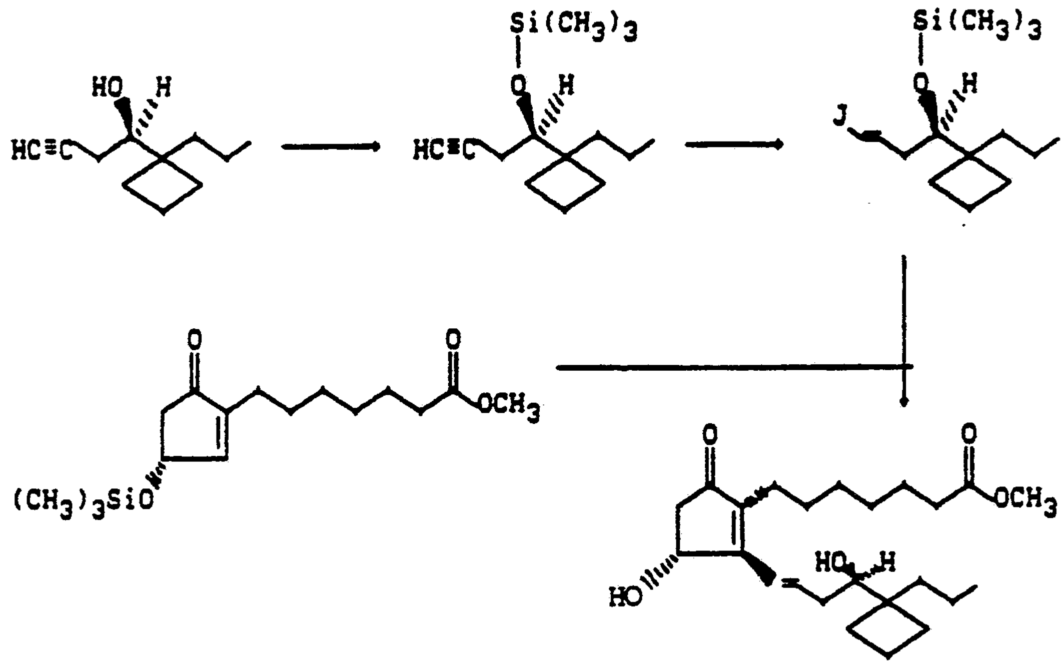

XXXXV

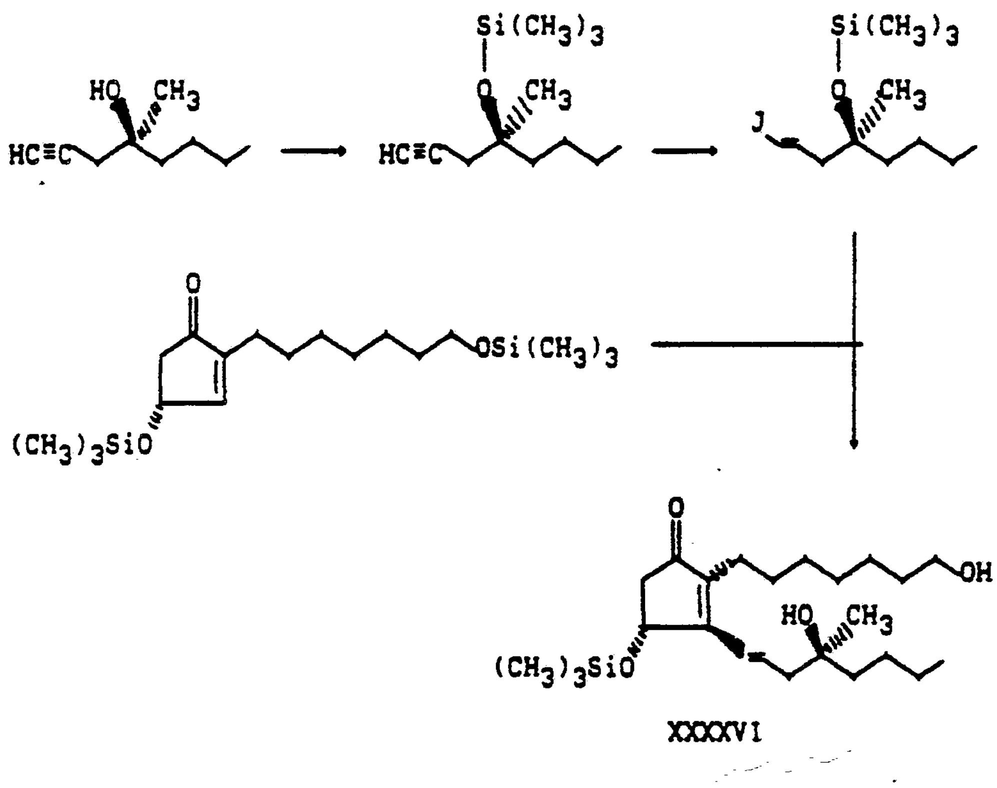

XXXXVI

<u>**Schema 4**</u>

## Beispiele

Beispiel 1

Zu einer Mischung von 166 g racemischem Octinol XXIV, 850 ml Methylenchlorid und 103 g Pyridin tropft man zwischen 0°C bis 10°C 227,5 g 1S-trans-3(2,2-Dichlorvinyl)-2,2-dimethylcyclopropancarbonsäurechlorid X und kocht 4 Stunden unter Rückfluß. Nach Abkühlen extrahiert man die Reaktionslösung zweimal mit jeweils 0,25 l 10 %iger Salzsäure und einmal mit 0,13 l gesättigter Natriumhydrogencarbonatlösung. Die organische Phase verdünnt man mit dem gleichen Volumen Petrolether und saugt über 0,3 kg Kieselgel ab. Man spült das Kieselgel mit 0,5 l Methylenchlorid/Petrolether (1:1) nach, dampft die vereinigten Eluate im Vakuum ein und erhält die diastereomeren 1S-Ester XXVI als 1:1-Gemisch.
Ausbeute: 332 g (84 % d.Th.) (Gehalt 90 %; GC)
$[\alpha]_{589}^{20}$ = +6,1° (c = 0, 7; $CHCl_3$)

Beispiel 2

Analog Beispiel 1 setzt man racemisches Octinol XXIV mit 1R-trans-3(2,2-Dichlorvinyl)-2,2-dimethylcyclopropancarbonsäurechlorid XI um und erhält die diastereomeren 1R-Ester XXXVI als 1:1-Gemisch.
Ausbeute: 336,9 g (85 % d.Th.) (Gehalt 90 %; GC)
$[\alpha]_{589}^{20}$ = -5,9° (c = 0, 64; $CHCl_3$)

Beispiel 3

Analog Beispiel 1 setzt man optisch aktives R-Octinol XXXIII mit 1S-trans-3(2,2-Dichlorvinyl)-2,2-dimethylcyclopropancarbonsäurechlorid X um und erhält den 1S-Ester XXIX als Kristallisat.
Ausbeute: 90,3 % d.Th.
Fp. 53-54°C
$[\alpha]_{365}^{20}$ = -7,3° (c = 1, $CHCl_3$)

Beispiel 4

Analog Beispiel 1 setzt man optisch aktives S-Octinol XXXIV mit 1R-trans-3(2,2-Dichlorvinyl)-2,2-dimethylcyclopropancarbonsäurechlorid XI um und erhält den 1R-Ester XXXX als Kristallisat.
Ausbeute: 91,1 % d.Th.
Fp. 52°C
$[\alpha]_{365}^{20}$ = +6,9° (c = 1, $CHCl_3$)

Beispiel 5

Analog Beispiel 1 setzt man optisch aktives R-Octinol XXXIII mit 1R-trans-3(2,2-Dichlorvinyl)-2,2-dimethylcyclopropancarbonsäurechlorid XI um und erhält den 1R-Ester XXXIX als Öl.
Ausbeute: 90,1 % d.Th.
$[\alpha]_{365}^{20}$ = -39,7° (c = 1, $CHCl_3$)

Beispiel 6

Analog Beispiel 1 setzt man optisch aktives S-Octinol XXXIV mit 1S-trans-3(2,2-Dichlorvinyl)-2,2-dimethylcyclopropancarbonsäurechlorid X um und erhält den 1S-Ester XXX als Öl.
Ausbeute: 86,1 % d.Th.
$[\alpha]_{365}^{20}$ = +38,1° (c = 1, $CHCl_3$)

Beispiel 7

Analog Beispiel 1 setzt man 140 g racemisches Alkinol XXIII mit 152 g Triethylamin, 12 g 4-Dimethylaminopyridin und 285 g 1S-trans-3 (2,2-Dichlorvinyl)-2,2-dimethylcyclopropancarbonsäurechlorid X ohne Lösungsmittel 15 Stunden lang bei 60°C um und erhält den diastereomeren Ester XXV als Öl.
Ausbeute: 361 g (77,6 % d.Th.)
$[\alpha]^{20}_{589}$ = -13,1° (c = 0,8; $CHCl_3$)

Beispiel 8

Analog Beispiel 7 setzt man racemisches Alkinol XXIII mit 1R-trans-3(2,2-Dichlorvinyl)-2,2-dimethylcyclopropancarbonsäurechlorid XI um und erhält den diastereomeren Ester XXXV als Öl.
Ausbeute: 356,8 g (76 % d.Th.)
$[\alpha]^{20}_{589}$ = +13,6° (c = 0,8; $CHCl_3$)

Beispiel 9

25 g R-Ester XXXVI aus Beispiel 2 werden über 1,3 kg Kieselgel (Si 60, 15-25 µm; Säulendurchmesser 8 cm; Fluß 110 ml/min) in Dioxan/Hexan (0,15/98,5) chromatographisch getrennt. Nach Abdampfen des Laufmittels erhält man die getrennten R-Ester.
Fraktion 1 (54-57,7 l) R-Ester XXXX als Kristallisat
Ausbeute: 5,5 g (22 % d.Th.), 95,4 % d.e. aus GC
Fraktion 2 (57,7-66,2 l) R-Ester XXXX und XXXIX als Öl
Ausbeute: 13 g (52 % d.Th.), 5 % d.e. aus GC
Fraktion 3 (66,2-76,2 l) R-Ester XXXIX als Öl
Ausbeute: 5,7 g (23 % d.Th.), 98,3 % d.e. aus GC

Beispiel 10

25 g S-Ester XXVI aus Beispiel 1 werden analog Beispiel 7 chromatographisch getrennt.
Fraktion 1 (53-56,8 l) S-Ester XXIX als Kristallisat
Ausbeute: 6,0 g (24 % d.Th.), 92,8 % d.e. aus GC
Fraktion 2 (56,8-68 l) S-Ester XXIX und XXX als Öl
Ausbeute: 14,1 g (56 % d.Th.), 6,5 % d.e. aus GC
Fraktion 3 (68-80,1 l) S-Ester XXX als Öl
Ausbeute: 4,6 g (18 % d.Th.), 97,3 % d.e. aus GC

Beispiel 11

100 g S-Ester XXVI aus Beispiel 1 werden per "Craig-Verteilung" (4200 Trennstufen, Cyclohexan/Nitromethan) getrennt.
Fraktion 1 S-Ester XXX als Öl
Ausbeute: 43 g (41 % d.Th.), 92 % d.e. aus GC
Fraktion 2 S-Ester XXIX als Kristallisat
Ausbeute: 44,7 g (42 % d.Th.), 90,2 % d.e. aus GC

Beispiel 12

1,55 kg S-Ester XXVI aus Beispiel 1 löst man in 3 l Methanol, kühlt auf 0°C, gibt ca. 10 g Impfkristalle hinzu und rührt über Nacht bei -20°C. Man saugt ab und rührt das feuchte Kristallisat bei -20°C mit 0,6 l Methanol 2 Stunden aus. Man saugt erneut ab und erhält dden S-Ester XXIX nach Trocknen im Vakuum bei Raumtemperatur als weißes Kristallisat.
Ausbeute: 590,2 g (38 %)

$[\alpha]^{20}_{365}$ = -7,22° (c = 1, $CHCl_3$)

Die S-Ester XXX erhält man nach Eindampfen der 1. Mutterlauge als ölige Flüssigkeit.

Ausbeute: 908 g (59 % d.Th.)

$[\alpha]^{20}_{365}$ = +24,8° (c = 1, $CHCl_3$)

Beispiel 13

Analog Beispiel 12 erhält man aus 1,55 kg R-Ester XXXVI aus Beispiel 2 den R-Ester XXXX als Kristallisat.

Ausbeute: 578,7 g (37 % d.Th.)

$[\alpha]^{20}_{365}$ = +7,08° (c = 1, $CHCl_3$)

Den R-Ester XXXIX erhält man nach Eindampfen der 1. Mutterlauge als ölige Flüssigkeit.

Ausbeute: 931 g (60 % d.Th.)

$[\alpha]^{20}_{365}$ = -27,79° (c = 1, $CHCl_3$)

Beispiel 14

Analog Beispiel 12 erhält man aus 360 g S-Ester XXV aus Beispiel 7 mit 1,4 l Methanol in 18 Stunden bei -70°C den S-Ester XXVII als Kristallisat.

Ausbeute: 101,8 g (35,9 % d.Th.)

$[\alpha]^{20}_{365}$ = -4,04° (c = 1; $CHCl_3$)

Beispiel 15

Analog Beispiel 14 erhält man aus dem R-Ester XXXV aus Beispiel 8 den R-Ester XXXVIII als Kristallisat.

Ausbeute: 89,5 g (31,6 % d.Th.)

$[\alpha]^{20}_{365}$ = +2,86° (c = 1, $CHCl_3$)

Beispiel 16

589 g kristallinen S-Ester XXIX aus Beispiel 12 fügt man zu einer Lösung aus 2,4 l Methanol und 140 g Natriumhydroxid und rührt die Lösung 6 Stunden bei 50°C. Nach Abkühlen dampft man das Lösungsmittel im Vakuum ab, nimmt den Eindampfrückstand in 1 l Wasser und 1 l Methylenchlorid auf, trennt die Phasen, extrahiert die wäßrige Phase zweimal mit je 0,5 l Methylenchlorid und wäscht die vereinigten organischen Phasen zweimal mit je 1 l Wasser. Nach Trocknen mit Natriumsulfat dampft man das Lösungsmittel im Vakuum ab und erhält das R-Octinol XXXIII durch Vakuumdestillation (Sdp. $_{13\ mbar}$ 99 bis 104°C).

Ausbeute: 251 g (91 % d.Th.)

$[\alpha]^{20}_{365}$ = -32,0° (c = 1, $CHCl_3$)

Die vereinigten wäßrigen Phasen stellt man mit konz. Salzsäure auf pH 1 und extrahiert zweimal mit je 0,5 l Methylenchlorid. Die vereinigten organischen Phasen trocknet man mit Natriumsulfat und erhält die 1S-trans-Permethrinsäure XXI nach Abdampfen des Lösungsmittels im Vakuum als gelbliches Kristallisat.

Ausbeute: 337 g (97 % d.Th.)

$[\alpha]^{20}_{365}$ = -114,4° (c = 1, $CHCl_3$)

Beispiel 17

Analog Beispiel 16 setzt man 321 g kristallinen R-Ester XXXX aus Beispiel 13 um und erhält das S-Octinol XXXIV.

Ausbeute: 137 g (91 % d.Th.)

$[\alpha]^{20}_{365}$ = +30,9° (c = 1, $CHCl_3$)

Weiterhin erhält man analog Beispiel 16 die 1R-trans-Permethrinsäure XXII.
Ausbeute: 182 g (95 % d.Th.)
$[\alpha]^{20}_{365}$ = +142° (c = 1, $CHCl_3$)

Beispiel 18

Analog Beispiel 16 setzt man 481 g öligen R-Ester XXXIX aus Beispiel 13 um und erhält das R-Octinol XXXIII.
Ausbeute: 193 g (86 % d.Th.)
$[\alpha]^{20}_{365}$ = 0 bis 22,1° (c = 1, $CHCl_3$)

Weiterhin erhält man analog Beispiel 16 die 1R-trans-Permethrinsäure XXII.
Ausbeute: 247 g (90 % d.Th.)
$[\alpha]^{20}_{365}$ = +143,1° (c = 1, $CHCl_3$)

Beispiel 19

S-Octinol XXXIV wurde analog Beispiel 4 mit 1R-trans-Permethrinsäurechlorid XI zu dem entsprechenden Ester XXXX umgesetzt. Diese Ester XXXX wurde analog Beispiel 16 wieder gespalten.

| Einsatz: | Ausbeute: |
|---|---|
| 20 g XXXIV | 17 g XXXIV |
| $[\alpha]^{20}_{365}$ =+26,8° (c=1, $CHCl_3$) | $[\alpha]^{20}_{365}$ = +26,7° (c=1,$CHCl_3$) |
| 27,6 g XXII für XI | 23,5 g XXIII |
| $[\alpha]^{20}_{365}$ =+141,7° (c=1, $CHCl_3$) | $[\alpha]^{20}_{365}$ = +140,1° (c=1,$CHCl_3$) |

Es findet also sowohl beim Octinol XXXIV als auch bei der Permethrinsäure XXII keine Racemisierung statt.

Beispiel 20

Analog Beispiel 16 setzt man 86,4 g S-Ester XXVII aus Beispiel 14 mit 22,6 g Natriumhydroxid und 375 ml Methanol 15 Stunden bei 50°C um und erhält das S-Alkinol XXXI als farblose Flüssigkeit.
Ausbeute: 25,1 g (69,7 % d.Th.)
$[\alpha]^{25}_{365}$ = +3,61° (C = 0,85; $CHCl_3$)

Beispiel 21

Analog Beispiel 20 setzt man 89,3 g R-Ester XXXVIII aus Beispiel 15 um und erhält das R-Alkinol XXXII als farblose Flüssigkeit.
Ausbeute: 26,7 g (73,4 % d.Th.)
$[\alpha]^{25}_{365}$ = -4,19° (c = 0,85; $CHCl_3$)

Beispiel 22

Zu einer Lösung von 16,6 g racemischem Octinol XXIV in 15,2 g Triethylamin und 1,2 g Dimethylaminopyridin tropft man zwischen 25 bis 45°C 12,8 g Acetanhydrid und läßt innerhalb von 1 Stunde auf Raumtemperatur abkühlen. Man gibt 100 ml Methylenchlorid hinzu und tropft unter Kühlung bei 20 bis 30°C 70 ml 2N-Salzsäure zu der Reaktionslösung. Man trennt die Phasen, wäscht die organische Phase zunächst mit 70 ml gesättigter Natriumhydrogencarbonatlösung und anschließend zweimal mit je 70 ml Wasser und trocknet mit Natriumsulfat. Nun dampft man das Lösungsmittel im Vakuum ab und erhält das racemische Acetyloctinol XXXXII nach Vakuumdestillation (Sdp. $_{14\ mbar}$ 110°C) als farblose Flüssigkeit.
Ausbeute: 18,8 g (90 % d.Th.)

Beispiel 23

146 g (0,701 Mol) racemisches Acetyloctinol XXXXII aus Beispiel 22 gibt man zu 30 l Phosphatpuffer pH 7 (Merck) und 100 g Lipase Typ VII (Sigma) und läßt 150 Stunden zwischen 30 und 33°C rühren, bis ca. die Hälfte des Acetats umgesetzt ist. Nach Abkühlen extrahiert man die Reaktionslösung zunächst mit 7,5 l Essigester und anschließend noch zweimal mit je 5 l Essigester und trocknet die vereinigten wäßrigen Phasen mit Natriumsulfat. Nach Abjdampfen des Lösungsmittels im Vakuum destilliert man den Rückstand über eine Füllkörperkolonne und erhält das S-Octinol XXXIV und das R-Acetyloctinol XXXXIV als farblose Flüssigkeiten.
Ausbeute: (S-Octinol XXXIV) 53,2 g (45,7 % d.Th.)
$[\alpha]^{20}_{365}$ = +27,9° (c = 1, $CHCl_3$)
Ausbeute (R-Acetyloctinol XXXXIV) 62 g (42,4 % d.Th.)

Beispiel 24

Analog Beispiel 16 werden 50 g R-Acetyloctinol aus Beispiel 23 mit 19,2 g Natriumhydroxid in Methanol zu dem R-Octinol XXXIII verseift.
Ausbeute: 35,9 g (91 % d.Th.)
$[\alpha]^{20}_{365}$ = -29,3° (c = 1, $CHCl_3$)

## Ansprüche

1. Verfahren zur Herstellung von optisch aktiven Octinolen der Formeln VIII und IX

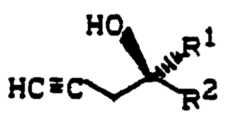
(VIII)

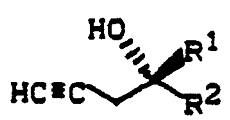
(IX)

in denen $R^1$ für Wasserstoff oder Methyl und $R^2$ für Alkyl steht, dadurch gekennzeichnet, daß man das racemische Octinol III

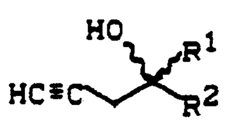
(III)

mit dem 1S-Permethrinsäurechlorid X

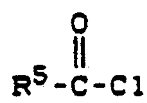

(X)

worin
$R^5$ für

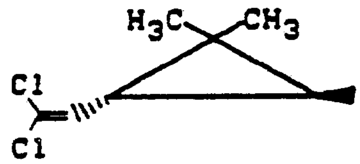

steht,
oder mit einem anderen aktivierten Derivat der 1S-Permethrinsäure zu den diastereomeren Permethrinsäureestern XIII

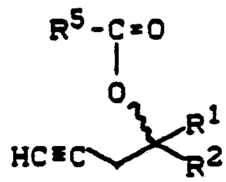

(XIII)

umsetzt, mittels präparativer Säulenchromatographie an Kieselgel und Flüssig-Flüssig-Extraktion in die einzelnen Diastereomeren XVI und XIX

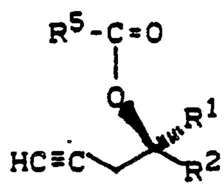

(XVI)

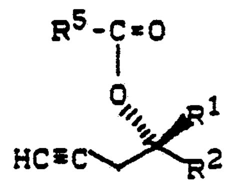

(XIX)

auftrennt oder das Estergemisch XIII durch selektive Kristallisation trennt, wobei der Ester XVI in hoher Diastereomerenreinheit auskristallisiert und der Ester XIX in hoher Reinheit aus der Mutterlauge gewonnen werden kann, und anschließend die optisch aktiven Ester alkalisch verseift und aus dem Reaktionsgemisch die optisch aktiven Octinole VIII bzw. IX nach üblichen Methoden isoliert.

2. Verfahren zur Herstellung von optisch aktiven Octinolen der Formeln VIII und IX

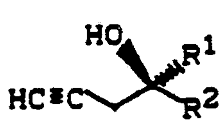

(VIII)

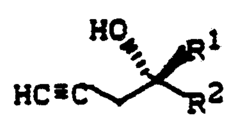

(IX)

in der $R^1$ für Wasserstoff oder Methyl und $R^2$ für Alkyl steht, dadurch gekennzeichnet, daß man das racemische Octinol III

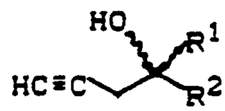

(III)

mit dem 1R-Permethrinsäurechlorid XI

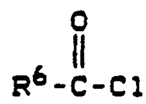

(XI)

worin
$R^6$ für

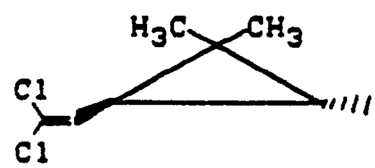

steht,
oder mit aktivierten Derivaten der 1R-Permethrinsäure zu den diastereomeren Permethrinsäureestern XIV

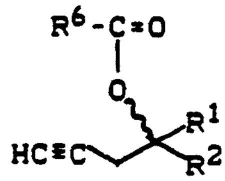

(XIV)

umsetzt, mittels präparativer Säulenchromatographie an Kieselgel und Flüssig-Flüssig-Extraktion in die einzelnen Diastereomeren XVII und XX

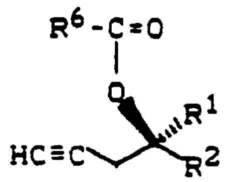

(XVII)

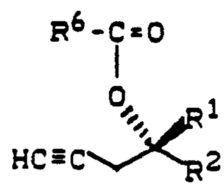

(XX)

auftrennt oder das Estergemisch XIV durch selektive Kristallisation trennt, wobei der Ester XX in hoher Diastereomerenreinheit auskristallisiert und der Ester XIII ebenfalls in hoher Reinheit aus der Mutterlauge gewonnen werden kann, und anschließend die optisch aktiven Ester alkalisch verseift und aus dem Reaktionsgemisch die optisch aktiven Octinole VIII bzw. IX nach üblichen Methoden isoliert.

3. Verfahren zur Herstellung von optisch aktiven Octinolen der Formeln VIII und IX

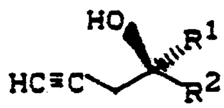

(VIII)

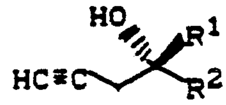

(IX)

in denen $R^1$ und $R^2$ die oben in Anspruch 1 angegebene Bedeutung haben, dadurch gekennzeichnet, daß man das racemische Octinol III

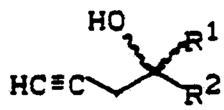

(III)

mit Anhydriden XII oder anderen aktivierten Carbonsäurederivaten in die Ester XV

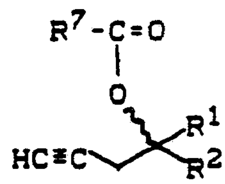

(XV)

worin $R^7$ für $CH_3$, $C_2H_5$, n-$C_3H_7$ oder $C_6H_5$ steht, überführt, mit Enzymen enantioselektiv hydrolysiert, wobei ein Gemisch aus dem optisch aktiven Ester XVII und dem optisch aktiven Octinol IV entsteht,

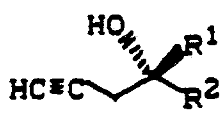

(IX)

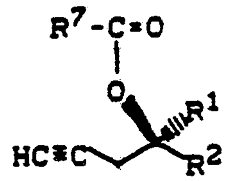

(XVIII)

das Gemisch chromatographisch oder durch Flüssig-Flüssig-Extraktion oder durch Destillation trennt und dem abgetrennten Ester XVIII alkalisch verseift und das optisch aktive Octinol VIII nach üblichen Methoden abtrennt.

4. Verbindungen der Formeln

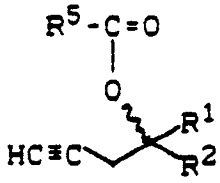
(XIII)

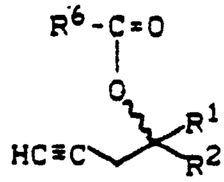
(XIV)

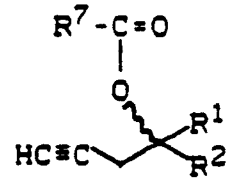
(XV)

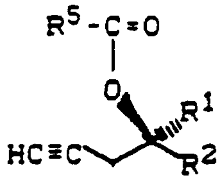
(XVI)

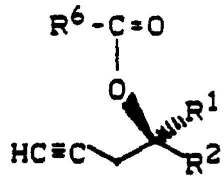
(XVII)

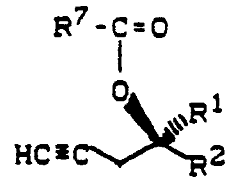
(XVIII)

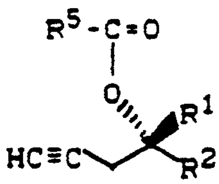
(XIX)

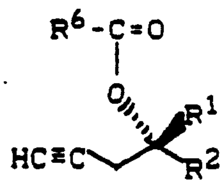
(XX)

worin
$R^5$ für

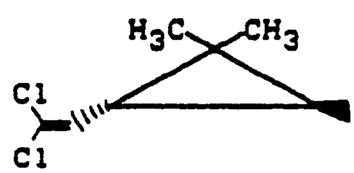

und
$R^6$ für

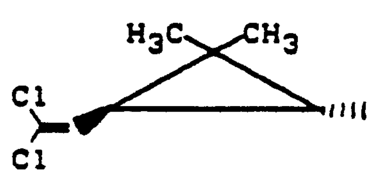

steht,
$R^7$ $-CH_3$, $-C_2H_5$, -n-$C_3H_7$, $-C_6H_5$ bedeutet, und
in denen $R^1$ für Wasserstoff oder Methyl und $R^2$ für Alkyl steht.

5. Verfahren zur Herstellung von optisch aktiven Octinolen der Formeln XXXIII und XXXIV

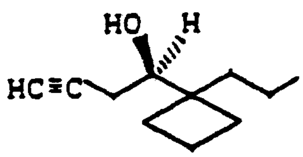

(XXXIII)

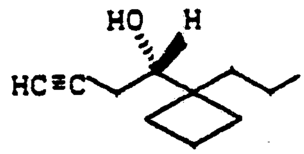

(XXXIV)

dadurch gekennzeichnet, daß man das racemische Octinol XXIV

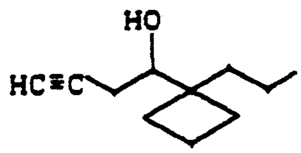

(XXIV)

mit den optisch aktiven Permethrinsäurechloriden X bzw. XI

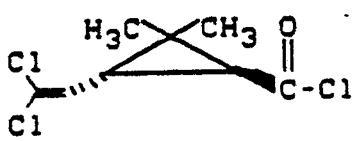

(X)

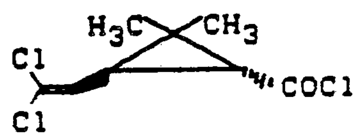

(XI)

zu den diastereomeren Permethrinsäureestern XXVI bzw. XXXVI

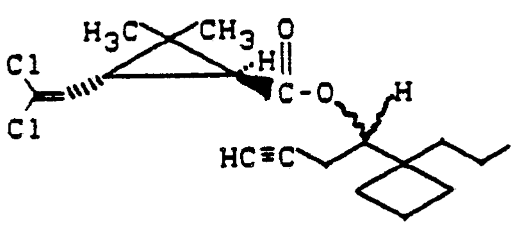

(XXVI)

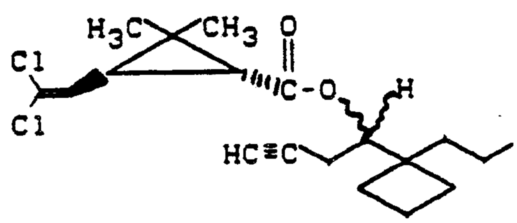

(XXXVI)

umsetzt, mittels präparativer Säulenchromatographie an Kieselgel und Flüssig-Flüssig-Extraktion in die einzelnen Diastereomeren XXIX und XXX bzw. XXXX und XXXIX

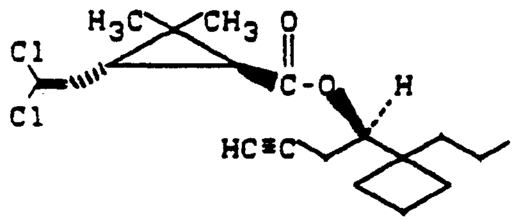

(XXIX) (Krist.)

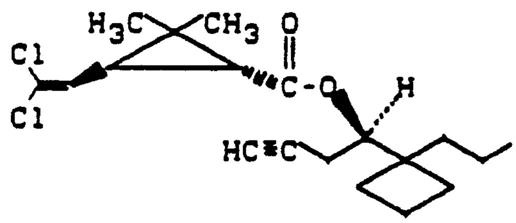

(XXXIX) (Öl)

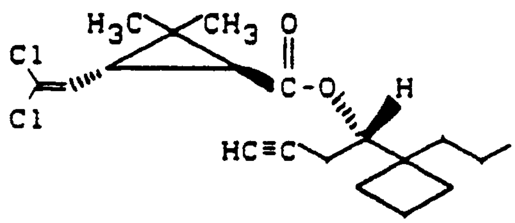

(XXX) (Öl)

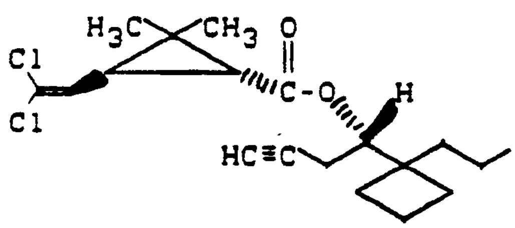

(XXXX) (Krist.)

auftrennt oder die Estergemische XXVI und XXXVI durch selektive Kristallisation trennt, wobei die Ester XXIX und XXXX in hoher Diastereomerenreinheit auskristallisieren und die Ester XXXIX und XXX ebenfalls in hoher Reinheit aus der Mutterlauge gewonnen werden können, und anschließend die optisch aktiven Ester alkalisch verseift und aus dem Reaktionsgemisch die optisch aktiven Octinole XXXIII bzw. XXXIV nach üblichen Methoden isoliert.

6. Verfahren zur Herstellung von optisch aktiven Octinolen der Formeln XXXIII und XXXIV

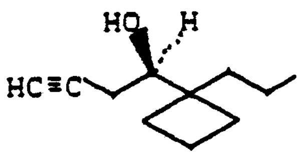

(XXXIII)

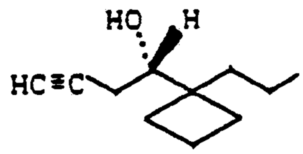

(XXXIV)

dadurch gekennzeichnet, daß man das racemische Octinol XXIV

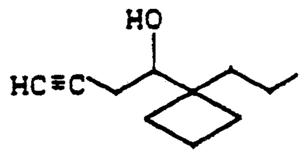

(XXIV)

mit Acetanhydrid oder Acetylchlorid in das Acetat XXXXII

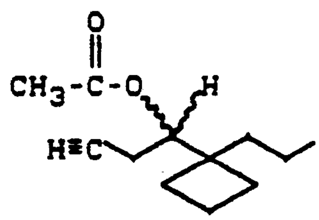

(XXXXII)

überführt, mit Enzymen enantioselektiv hydrolysiert, wobei ein Gemisch aus dem optisch aktiven Acetat XXXXIV und dem optisch aktiven Octinol XXXIV entsteht,

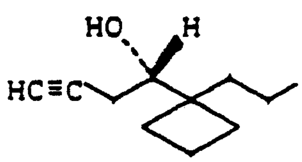

(XXXIV)

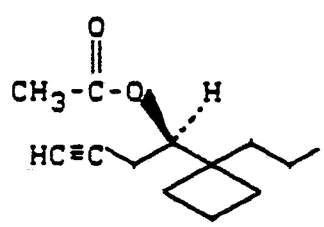

(XXXXIV)

das Gemisch chromatographisch oder durch Flüssig-Flüssig-Extraktion oder durch Destillation trennt und das abgetrennte Acetat alkalisch verseift und das optisch aktive Octinol nach üblichen Methoden abtrennt.

7. Verbindungen aus der Gruppe, bestehend aus

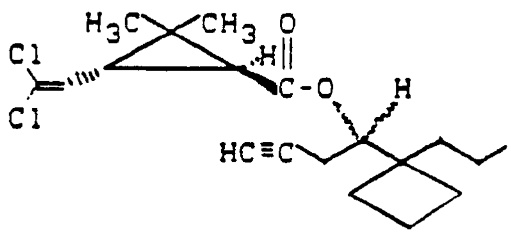
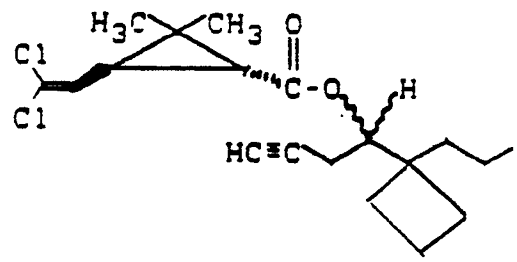

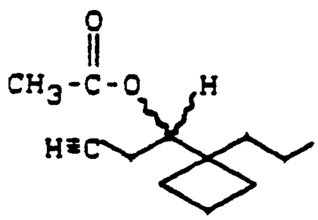
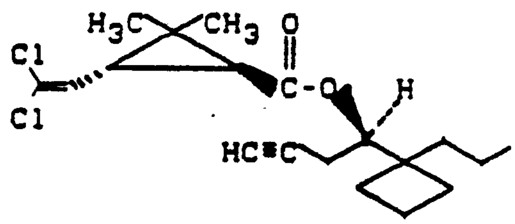

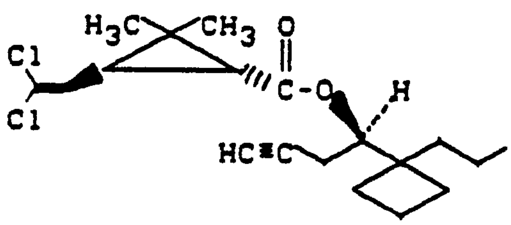
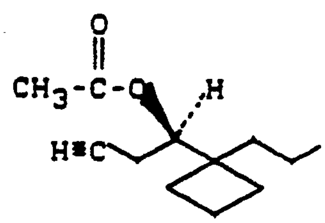

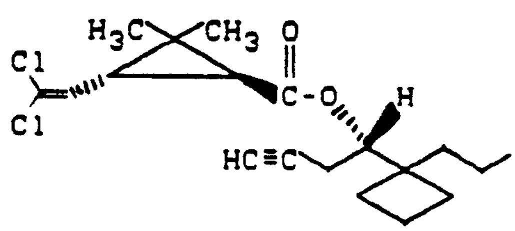

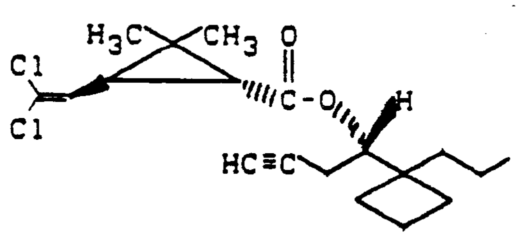

8. Verwendung der Verbindungen gemäß Anspruch 7 bei der Herstellung von Prostaglandinen.

9. Verwendung von 4R-oder 4S-5,5-Propano octin-4-ol, erhalten nach dem Verfahren gemäß Anspruch 5, zur Herstellung von Prostaglandinen.

10. Verwendung von 4R-oder 4S-5,5-Propano octin-4-ol, erhalten nach dem Verfahren gemäß Anspruch 6, zur Herstellung von Prostaglandinen.

11. Verfahren zur Herstellung von optisch aktiven Octinolen der Formeln XXXI und XXXII

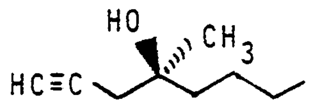

(XXXI)

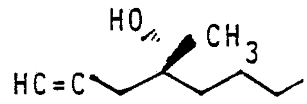

(XXXII)

dadurch gekennzeichnet, daß man das racemische Octinol XXIII

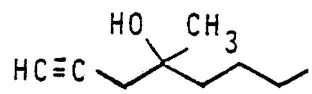

(XXIII)

mit den optisch aktiven Permethrinsäurechloriden X bzw. XI

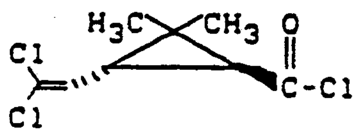

(X)

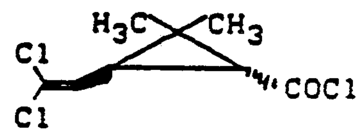

(XI)

zu den diastereomeren Permethrinsäureestern XXV bzw. XXXV

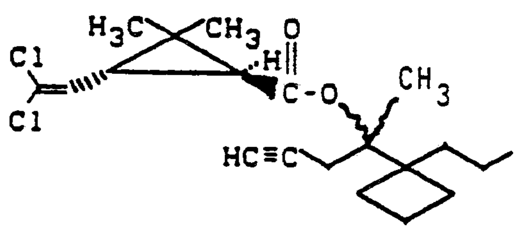

(XXV)

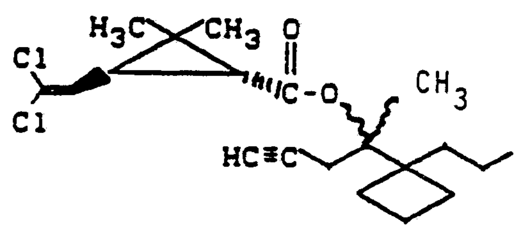

(XXXV)

umsetzt, mittels präparativer Säulenchromatographie an Kieselgel und Flüssig-Flüssig-Extraktion in die einzelenen Diastereomeren XXIX und XXX bzw. XXXX und XXXIX

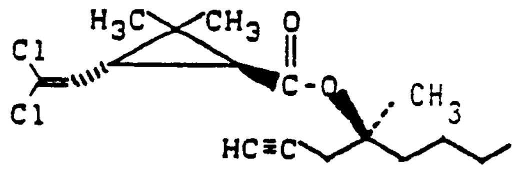

(XXVII) (Krist.)

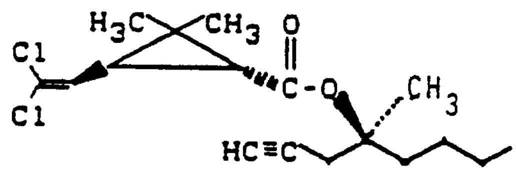

(XXXVII) (Öl)

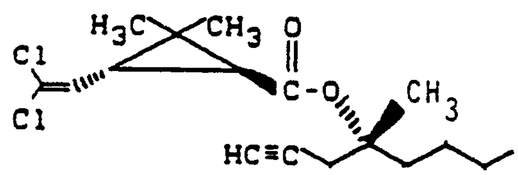

(XXVIII) (Öl)

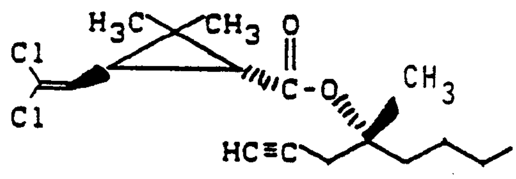

(XXXVIII) (Krist.)

auftrennt oder die Estergemische XXV und XXXV durch selektive Kristallisation trennt, wobei die Ester XXVII und XXXVIII in hoher Diastereomerenreinheit auskristallisieren und die Ester XXXVII und XXVIII ebenfalls in hoher Reinheit aus der Mutterlauge gewonnen werden können, und anschließend die optisch aktiven Ester alkalisch verseift und aus dem Reaktionsgemisch die optisch aktiven Octinole XXXI und XXXII nach üblichen Methoden isoliert.

12. Verfahren zur Herstellung von optisch aktiven Octinolen der Formeln XXXI und XXXII

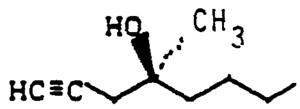 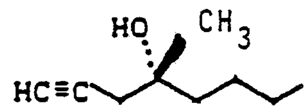

(XXXI) (XXXII)

dadurch gekennzeichnet, daß man das racemische Octinol XXIII

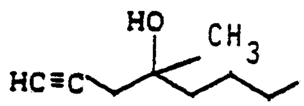

(XXIII)

mit Acetanhydrid oder Acetylchlorid in das Acetat XXXXI

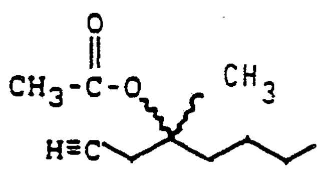

(XXXXI)

überführt, mit Enzymen enantioselektiv hydrolysiert, wobei ein Gemisch aus dem optisch aktiven Acetat XXXXIII und dem optisch aktiven Octinol XXXII entsteht,

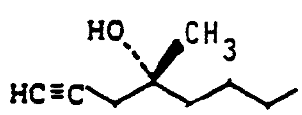 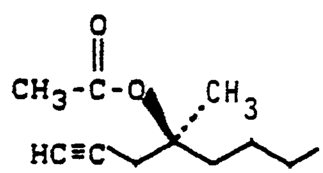

(XXXII) (XXXXIII)

das Gemisch chromatographisch oder durch Flüssig-Flüssig-Extraktion oder durch Destillation trennt und das abgetrennte Acetat alkalisch verseift und das optisch aktive Octinol nach üblichen Methoden abtrennt.

13. Verbindungen aus der Gruppe, bestehend aus

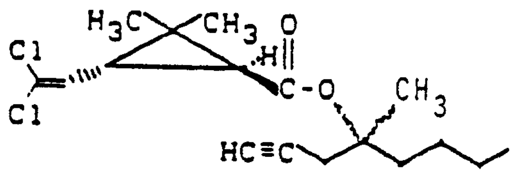

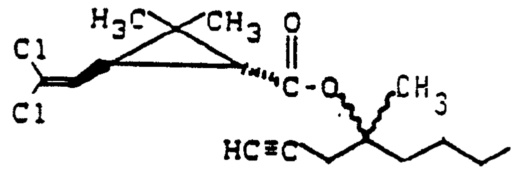

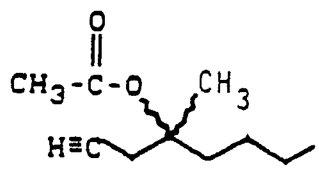

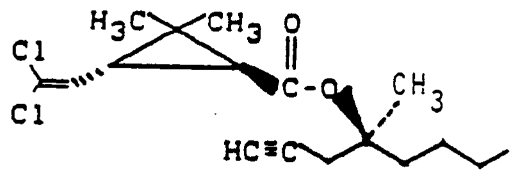

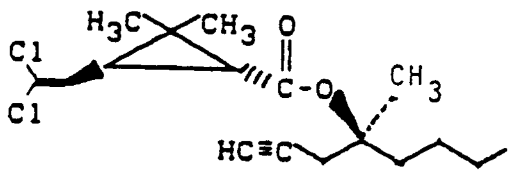

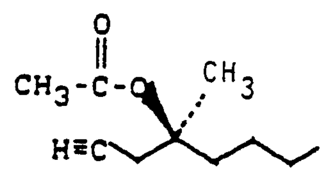

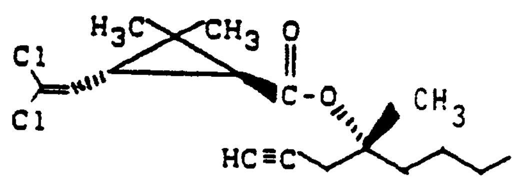

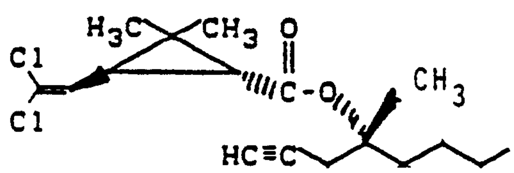

14. Verwendung der Verbindungen gemäß Anspruch 13 bei der Herstellung von Prostaglandinen.

15. Verwendung von 4R-oder 4S-4-Methoyloct-4-inol, erhalten nach dem Verfahren gemäß Anspruch 11, zur Herstellung von Prostaglandinen.

16. Verwendung von 4R-oder 4S-4-Methyloct-4-inol, erhalten nach dem Verfahren gemäß Anspruch 12, zur Herstellung von Prostaglandinen.

Europäisches Patentamt

# EUROPÄISCHER RECHERCHENBERICHT

Nummer der Anmeldung

EP 88 10 7063

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | JOURNAL OF ORGANIC CHEMISTRY, Bd. 42, no. 9, 29. April 1977, Seiten 1659-1660, American Chemical Society, Columbus, Ohio, US; W.J. MCGAHREN et al.: "Enzymatic and chemical resolution of 1-Octyn-4-ol"<br>* der ganze Artikel * | 1-4 | C 07 C 29/92<br>C 07 C 33/05<br>C 07 C 33/042<br>C 07 C 33/12<br>C 07 C 33/14<br>C 12 P 41/00<br>C 07 C 69/65 |
| Y | EP-A-0 022 972 (BAYER AG)<br>* Seite 7, Zeile 13--19 * | 1,2,4 | |
| Y | EP-A-0 149 674 (SUMITOMO CHEMICL CO. LTD.)<br>* Anspruch * | 1,3,4 | |
| A | EP-A-0 148 272 (SUMITOME CHEMICAL CO.)<br>* Anspruch 1 * | 3 | |
| A | US-A-4 132 738 (H.C. KLUENDER)<br>* Spalte 43, Zeilen 54-65; Spalte 31, Zeile 2; Spalte 27, Zeile 7; Spalte 29,Zeile 34 * | 1,5 | |

RECHERCHIERTE SACHGEBIETE (Int. Cl.4)

C 12 P 41/00
C 07 C 33/00
C 07 C 29/00
C 07 C 69/00

Der vorliegende Recherchenbericht wurde für alle Patentansprüche erstellt

| Recherchenort | Abschlußdatum der Recherche | Prüfer |
|---|---|---|
| BERLIN | 22-07-1988 | PROBERT C.L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratür

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)